# EUROPEAN PATENT SPECIFICATION

(11) **EP 1 304 978 B1**
(45) Date of publication and mention of the grant of the patent: **03.10.2007**
(21) Application number: 01985666.5
(22) Date of filing: 31.07.2001
(51) Int. Cl.: A61C 19/06

(54) **PERSONAL ORAL HYGIENE COMPOSITION AND DEVICE**
MITTEL UND VORRICHTUNG ZUR PERSÖNLICHEN ORALEN HYGIENE
DISPOSITIF ET COMPOSITION D'HYGIENE BUCCO-DENTAIRE

(30) Priority: 31.07.2000 NZ 50608800; 04.07.2001 NZ 51276001
(43) Date of publication of application: 02.05.2003
(73) Proprietor: Alexander, Carl Ernest, High Halden, Kent TN26 3JB (GB)
(72) Inventor: Alexander, Carl Ernest, High Halden, Kent TN26 3JB (GB)
(74) Representative: Laitinen, Pauli Sakari
(86) International application number: PCT/IB2001/002772
(87) International publication number: WO 2002/026078

(56) References cited:
- DE-A1- 3 429 619
- DE-A1- 4 238 421

## Description

### FIELD

This invention relates to a portable oral hygiene device and delivery systems and more particularly to toothpastes (or dentifrices) in an externally dry / free-flow form adapted for storage within a specially adapted toothbrush handle or a dispenser, for release and use as required.

### BACKGROUND

Ordinary toothpaste (typically a suspension of abrasive, salts, flavours, saccharin, etc) as supplied within a deformable container is reasonably convenient, but messy if it escapes (as in one's luggage or handbag), and is hard to dispense in simple unit amounts. (There is a saying that you can't put the toothpaste back in the tube). The only suitable container appears to be an enclosed deformable toothpaste tube. There are a number of occasions when just a small amount of toothpaste may be needed for a short trip. A toothbrush including convenient, reliable storage of a few brushings worth of a toothpaste equivalent would be useful for many of those people whose life takes them outside the home.

The problem to be solved might be described as "provision of a convenient, compact oral/dental hygiene device". Particular problems include "for travel or unpredictable possibly "social emergency" use by a person" and offering (for children) "raised attractiveness of the product". A social emergency might occur when about to meet a desirable person or a business contact but being aware that one's teeth are dirty and giving off offensive odours. A very large number of toothbrushes including inbuilt toothpaste dispensing apparatus are known in the patent literature but nevertheless few seem to have reached the market. No new toothpaste analogue has appeared for many years, apart from additives to conventional toothpaste such as fluoride, whiteners, or phosphates. Searches in even the trade mark databases (goods fields) and general Internet databases have failed to find any citations for a new form of toothpaste of the type to be described herein.

Environmental advantages exist in reducing the need for toothpaste tubes thereby reducing land fill problems and rubbish disposal. Less waste and less residue is likely if an alternative packaging process and appropriate delivery system can be developed that optimises the amount of a dental hygiene composition taken by a person at each occasion.

Spread of diseases by oral transmission at social encounters is becoming increasingly recognised; meningitis and tuberculosis for example - and there is a need to this oral hygiene problem to be addressed.

Another problem to be solved is that of encouraging children to brush their teeth on a regular basis (even when away from home). An alternative convenient and child-friendly preparation may be of considerable help in terms of paediatric dentistry and minimisation of public health problems in later life.

Document DE 42 38 421 discloses a system for facilitating the practice of oral and dental hygiene according to the preamble of Claim 1.

### OBJECT

It is an object of this invention to provide an improved system for delivery of dental hygiene; based on a dental hygiene composition and with optional apparatus for supply thereof, or at least to provide the public with a useful choice.

### STATEMENT OF INVENTION

In a first broad aspect the invention provides a system for facilitating the practice of oral and dental hygiene, wherein the system provides a dental hygiene composition for use when cleaning the teeth in the form of a plurality of unitary beads (alias capsules, tablets, lumps, etc); the dental hygiene composition within each bead comprising a paste, the paste being contained within a wall having a dry exterior, each bead has a wall strength sufficiently low to allow the contents to escape when the bead is caused to rupture by being pressed against the teeth or on to a toothbrush, and wherein the system also includes means for storing and dispensing said beads on demand, so that a person can practice dental hygiene more conveniently.

Preferably each bead contains about sufficient composition to supply a single procedure for brushing the teeth, so that a more convenient form of storage and dispensing of toothpaste is provided.

Preferably each bead wall is capable of maintaining a separation of the moist capsule interior from the dry environment surrounding the or each capsule.

Preferably the wall of each bead is made by a locally applied process (such as local heat) acting on the substance of the toothpaste.

Preferred locally applied processes include heating, drying, extrusion, dusting, moulding, varnishing, and coating treatment of the toothpaste.

Optionally the processes include admixture of additional materials hence optionally the or each capsule wall includes further materials capable of separating the moist capsule interior from the dry environment surrounding the or each capsule.

A further alternative is that the wall of each bead is made by by a process including application of a sealing substance over the exterior of a bead of the dental hygiene composition.

Preferably the wall of each bead is forced into a desired shape by a moulding process.

More preferably an amount of the composition is pressed into a die having a cavity of a desired shape, so that on subsequent ejection the or each bead has a specified shape.

Optionally, the method includes the prior step of coating the interior surface of the die with a wall forming agent or co-forming agent.

Alternatively, each bead is made by inserting a dental hygiene composition into a previously constructed container such as a starch or gelatine capsule.

Optionally the surfaces of the die are dusted prior to an extrusion moulding process using a dust of a material capable of assisting in the formation of a capsule wall.

In another option the die is heated and the exterior of the bead is seared by contact with the walls of the cavity for a controlled period so that on ejection the or each bead has a specified shape and the skin is rendered effectively impervious to water.

Preferably the beads are capable of extended periods of storage prior to use.

In a further method the steps include the steps of formulating a paste from selected ingredients, extruding the paste into a space, chopping the extruded paste at intervals into a series of beads, and allowing the separated beads to fall against a current of hot gas of a controlled temperature for a controlled time, then collecting the beads and packing them for use.

Preferably the current of hot air has a flow rate and upwards component of direction sufficient to grade the beads by size (if the current of air can almost support the standard bead size, the resultant grading action may be used to reject under-sized and oversized beads).

Alternatively, the chopped-off paste may fall down a shaft filled with hot air, as for the manufacture of lead shot involving a long vertical drop.

In another related aspect the invention provides a complete kit for personal application of dental hygiene, including beads of a dental hygiene composition, dispensing means therefor, and, in addition, brush application means, which kit is provided for sale as a set of items or separately.

In a yet another related aspect the invention provides a method for making a bead of toothpaste, wherein a gelatin coating (or the like) is applied by dipping extruded beads of paste into a solution and then drying.

Alternatively a solution may be sprayed on to beads while they are suspended in a current of air or similar.

In a still further related aspect the invention provides a method for making a bead of toothpaste, wherein a starch may be applied for example by dipping the bead in starch powder.

In an even further related aspect the invention provides a method for making a bead of toothpaste, wherein an acceptable volatile solvent (either miscible with water and with wax, or miscible with wax and briefly dried outer coatings) carries a wax or the like into the outer surface of the bead to act as a water-repellent binder. Preferably not enough wax is used to form a detectable residue or "skin" in the user's mouth. Outer skins (if any) will be of a substance suitable, and safe, for rinsing away in the usual manner.

Preferably each bead is decoratively shaped in order to appeal in particular to a child, so that the child is more likely to make use of the bead for oral and dental hygiene.

Also, preferably each bead is coloured in order to appeal in particular to a child, so that the child is more likely to make use of the bead for oral and dental hygiene.

Further, each bead is preferably flavoured in order to appeal in particular to a child, so that the child is more likely to make use of the bead for oral and dental hygiene.

Optionally the capsules/beads may include an optionally encapsulated gas generating substance.

In a second broad aspect the invention provides means for storing and dispensing beads of a composition as previously described in this section, wherein the means comprises a toothbrush including at least one resealable cavity each capable of storing a plurality of discrete items, so that both the composition and brush application means for applying the composition to one's teeth are kept together.

In a related aspect, the toothbrush includes two resealable cavities, so that at least one cavity is available for storage of an oral hygiene commodity other than a dental hygiene composition.

Another means comprises a free-standing dispenser adapted for the storage and dispensing of said encapsulated beads or capsules.

An alternative means comprises a wall-mounted dispenser adapted for the storage and dispensing of said encapsulated beads or capsules.

In a third broad aspect the invention provides a system for delivery of dental hygiene, wherein a manufactured dental hygiene composition or toothpaste in the form of encapsulated beads or capsules is obtained by a consumer from a point of sale, stored prior to use within a dispenser adapted for the storage and presentation of said encapsulated beads or capsules, and is used from time to time by the or any consumer as individual encapsulated beads or capsules of toothpaste, so that the system employs the beads or capsules in order to facilitate the application of dental hygiene.

In a related aspect the system for delivery of dental hygiene (including encapsulated beads or capsules and application means (such as a toothbrush) is provided as a set of items at a point of sale, in a readily portable format for use anywhere, home, office, holiday etc or a dispensing system for domestic or similar use.

### PREFERRED EMBODIMENT

The description of the invention to be provided herein is given purely by way of example and is not to be taken in any way as limiting the scope or extent of the invention.

### DRAWINGS

- Fig 1:: shows in outline or surface view several embodiments of capsules of encapsulated toothpaste .
- Fig 2:: shows a basic dispenser for encapsulated toothpaste.
- Fig 3:: shows another version of a dispenser.
- Fig 4:: shows a novelty container for encapsulated toothpaste.
- Fig 5:: shows a wall-mounted container for encapsulated toothpaste.
- Fig 6:: shows an example retail pack of encapsulated toothpaste and a toothbrush.
- Fig 7:: shows an example toothbrush with a storage cavity within its handle according to the invention, ready for use.
- Fig 7B:: shows that example toothbrush according to the invention, packed away.
- Fig 8:: shows a toothbrush with two storage cavities within its handle.
- Fig 9:: shows another toothbrush according to the invention in an example retail pack, with spare encapsulated toothpaste supplies and several exchangeable heads.

### INTRODUCTION

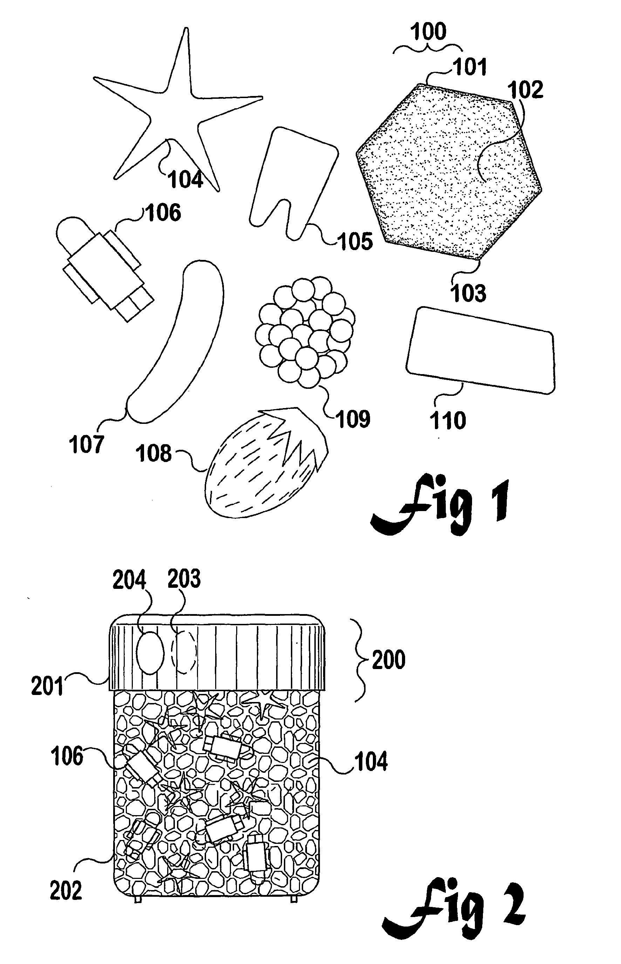

This invention provides a form of dental hygiene preparation in which a composition broadly similar to that of conventional toothpaste as obtained from a tube is provided within a plurality of individual portions, which may be called "capsules", "globules", "tablets", "beads", "pellets", and "packets" to give a few examples of alternative terms. One point of this invention is convenience: each capsule or portion may be dimensioned so that it will hold about the right amount of toothpaste for an average tooth-cleaning procedure. Capsules can be made in almost any consistency, flavour, colour, shape, or size, and some examples are shown in Fig 1. Capsules of various sizes (capacities) may be made available for small children, larger children, or adults. Even the existence of a choice is likely to promote the frequency of cleaning of teeth (providing a choice being a well-known subterfuge when causing children to comply with their parents' requests). Capsules and their dispensers can be made far more attractive to children.

It will be appreciated by a reader skilled in the art that the scope of this invention extends to all forms of toothpaste when provided as individual portions suitable for single use.

Note that the composition is dry granules and/or free-flowing, no longer needing the special containment provided by a toothpaste tube, so that a person can decant some capsules from a large container into a small container as for example when travelling. (The "small container" may for example be within a toothbrush handle). A further point is that the waste disposal problem caused by discarded toothpaste tubes in particular is abolished. (There may be 24-36 million discarded tubes per year in even a small country such as New Zealand; population 3.8 million).

### EXAMPLE 1 (Encapsulated toothpaste pellets)

### STRUCTURE

Fig 1 includes a section through one bead or capsule 100, where the interior 102 is separated from the external environment by a manufactured wall 101. This section shows a hexagonal outline as a result of that profile being given to the extrusion nozzle so that resulting beads tend not to roll, having corners 103. Other outlines of capsules are illustrated in Fig 1, where 104 is a star (child's night-time cleaning), 105 is a tooth shape (for serious children), 106 is a tiny robot, 107 is a jelly-bean shape, 108 is a strawberry (that could be made in a two-colour form with a green cap and a pink/red body) 109 is a raspberry (having a different internal flavour from 108) and 110 is a straightforward cube for economy.

Capsule walls may for example be made of: (1) dried toothpaste substance itself, (2) gelatin, (3) a light crusty paste including extra material, perhaps similar in consistency to that of marzipan, (4) a starch, or (5) for example a waxy impregation. Ordinary toothpaste as made is quite thick and requires moisteners or lubricants in order to be packed in, and then extruded from, a tube.

The actual toothpaste held within each bead may be of a gel or of a paste consistency. The wall of the bead should be able to fully contain the contents during storage, and then preferably (though not essentially) be capable of dissolving within the mouth leaving no solid residue. For the first wall option, certain thickeners in particular have the property of being convertible into a relatively stable impervious material when heated and dried. Avoidance of coatings is preferred in order to avoid foreign bodies (shells and crusts) appearing within the user's mouth, and to minimise manufacturing costs and process complexity. Nevertheless we shall consider some.

One possible coating material is starch or the like, capable of being formed into a firm outer shell that can be burst by pressing onto the teeth, crushing between the teeth, or pressing on to the bristles of the brush. Sucrose and other sugar-based materials are best avoided in toothpastes, although addition of saccharin or other artificial sweeteners may be useful. Food-grade dyes may be used to make the beads more visually interesting and any one dye may be linked to a certain variant of flavour in the same manner that red jellybeans have a raspberry flavour, and so on.

The size of beads is preferably adjusted so that one bead is sufficient for one tooth brushing procedure; optionally half a "dose" (to allow children to mix flavours) but preferably not much more than that for a full procedure, because of consequent waste.

The shape of beads can be altered to suit the market sector being catered to. For example children will be attracted by shapes such as stars, robots, flowers, television characters and the like. Adults may simply want the most compact shape possible. Shapes having a spherical section may be less desirable in that they can roll around, so triangular or hexagonal cross-sections may be preferred.

### MANUFACTURE

### 1. Solely heat-dried exteriors.

Manufacture of capsules formed solely of heat-dried toothpaste substance itself may proceed as follows: a paste including a selection of the usual toothpaste components such as a mineral abrasive, a detergent, and a flavouring agent such as a peppermint or a colour - related flavour such as strawberry or raspberry, as are known in the art, is extruded through an orifice of controlled shape into an air-filled space, chopped off at intervals, and may then be allowed to fall against a current of hot air of a predetermined temperature, and predetermined dryness, for a predetermined time. The current may be strong enough to almost support the standard bead size, in which case the inherent and convenient grading action of the air current (if constant) may be used to reject under-sized and oversized beads. Alternatively, the chopped-off paste may fall down a shaft filled with hot air, using the principle of the manufacture of lead shot involving a long vertical drop. The desired drying process should be effective only on the outer skin or crust. Preferably the interior remains moist. Beads made in this process are likely to be of variable shapes.

Another manufacturing method may resemble the process of injection moulding for shapes in plastics, wherein the paste is extruded under pressure into a die having a cavity of a desired shape. In this case there is more latitude for a variety of flavouring, in accordance with the shape of the resulting bead(s). The resulting bead becomes fixed in a certain shape after moulding as a result of one or more of the following processes possibly used in combination:
(1) effects of the pressure alone, pushing the toothpaste against the walls of the mould,
(2) drying; the cavity had porous walls which drained off fluid from the exterior, so that the exterior surface became dried more quickly,
(3) heat; the cavity was heated, and the heat seared the exterior surfaces of the capsule so that they were rendered effective as sealing walls,
(4) a coating; the cavity had been dusted with a powder before closing and extrusion. The powder may be a water-absorbing powder, a reactive powder capable of reacting to form a sealed surface (and might include at least one encapsulated material ruptured by the pressure of extrusion), or a meltable powder such as a wax.

Additional sealing may be employed, in particular where the product is to be stored in or shipped to humid areas.

### 2. Additional sealing materials.

The objective of drying is to moisture-seal the exterior of each bead. In some cases supplementary sealing, by the use of a gelatin coating (or the like) may involve dipping extruded beads of paste with a solution, or otherwise coating the beads (or spraying a solution on to beads while they are suspended in a current of air) and then drying. A coating may be preferred for beads of toothpaste intended for use in tropical or humid climates. Materials such as methyl cellulose, acacia (gum arabic), tragacanth, or the like may be useful. Other crusts such as starch may be applied in a similar way. Beeswax, a natural wax, or more preferably small amounts of higher melting point paraffin waxes may be used. An acceptable volatile solvent (miscible with water and with wax, or miscible with wax and briefly dried outer coatings) for carrying a wax or the like may be used to apply sufficient wax to the outer surface of the bead to act as a water-repellent binder, but not enough to form a detectable "skin" in the user's mouth. Critical-point liquefied carbon dioxide may be one acceptable solvent.

A water soluble (from the outside) yet water impermeable (from the inside) "disappearing-in-use" bead shell is ideal for this application. It may be that the presence of one or more insoluble remnants of a shell in one's mouth is more acceptable during a teeth cleaning operation than at other times. Generally, spitting into a receptacle is part of cleaning one's teeth. Nevertheless, it may be preferable to have the shell made of a material, such as one based on starch, capable of being attacked by salivary enzymes and so completely disappearing. Potato starch based capsules are a commercial product. It may even be acceptable to rely on other gut enzymes to complete the digestion process.

### 3. Taste-attractive crusts or skins.

Although we have preferred to create "disappearing wall material" which in effect deletes itself from the perceptible environment there are precedents for instead creating an attractive wall, at least in terms of taste. Consider the skin of a deep-fried potato chip, or the exterior of a "Jaffa" (TM) orange-coated chocolate ball. The skin of each capsule could hold the majority of the flavour deposited in each capsule.

### 4. Fully dried interior contents.

Optionally each bead may hold a dry material including a health-compatible gas-producing effervescent material such as "sherbet" (sodium potassium tartrate with sodium bicarbonate) or grains of "Space Dust" (TM) in order to excite a child's interest and possibly also in order to aid in "breaking up" the dry material. Fully dried beads may be manufactured using the technology of and optionally additives well known to the pharmaceutical tablet-making industries; possibly preferring options leading to a low to medium hardness.

### 5. Pressed or cut from a stiff paste.

During manufacture, a toothpaste composition may be formed into a stiff mass having a consistency rather like rolled-out pastry. This paste may be rolled out into a sheet and then cut, stamped, or otherwise formed into individual portions, which are then optionally dried, and/or coated, and/or wrapped. Indicia may be pressed into the surface of each piece. The paste may be extruded and dried (as previously described) or it may be extruded, coated, and heat-treated.

### USE

One or perhaps more beads are selected for use, removed from a container or dispenser and broken open either by finger pressure, or pressing the bead on to the bristles of a brush, or against the teeth, or may be bitten. Once the internally stored toothpaste is released into the mouth, a conventional toothbrush (which may be electric) is used in the normal manner.

### EXAMPLE 2: Dispensers.

Several convenient kinds of dispenser have been developed to serve as part of the invention as described herein (although the skilled reader should realise that a very large number of other dispensers may also be developed, but nevertheless fall within the scope of the invention). Representative examples of dispensers are shown in Figs 2 to 5.

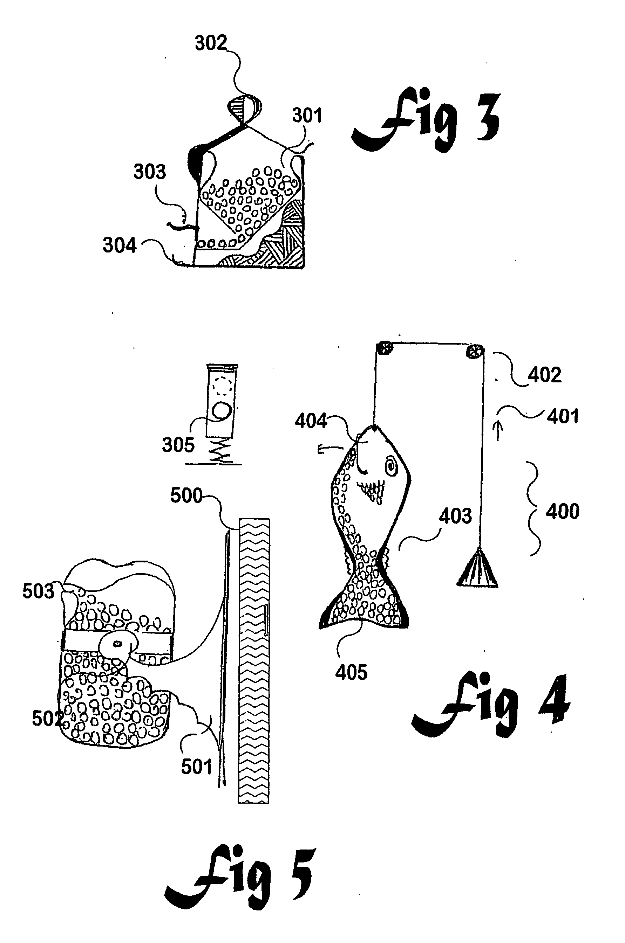

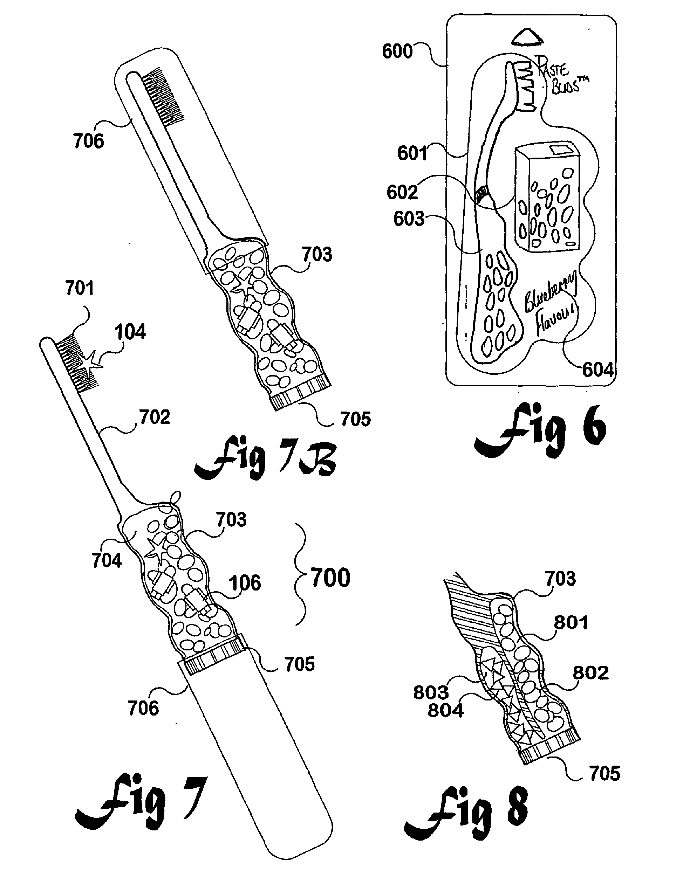

One large dispenser (e.g. fig 2 or fig 5) in the bathroom may serve an entire family. The simple dispenser of fig 2 includes several components: a container body 202 and a threaded (screw) lid 201. On the side of the lid is a hole 204 capable of being matched with a hole 203 within the top of the container body, for shaking out one or more capsules. The filling of beads is indicated at 104 - with visible robots 106 for example. The dispenser may be refilled from time to time with a retail pack of capsules, such as a plastic bag or even a paper bag of capsules. Fig 3 shows another conventional dispenser with a lid 302. A spring-loaded lever 303 when pressed aligns two holes so that a capsule or bead previously stored within the mass 301 emerges through hole 305 within a plunger assembly on to a collection plate 304. In fig 5, the entire dispenser is mounted from a wall 500 by means of an arm 501 supporting a pivot 502, so that on tilting, one or more capsules emerge at the orifice 503. Alternative dispensers may resemble, and may even be used as, toys for greater appeal to children. Fig 4 shows a "jiggling fish" version 400 in which the body of the fish serves to hold a mass of capsules 405 for dispensing through the mouth 404 when that is pulled forward. On release the weight at the end of the cord 401, acting around pulleys 402 causes the fish to revert to an upward attitude as shown here. Smaller dispensers may be scaled down in order to fit into handbags or may even become part of toothbrushes - such as handles.

### EXAMPLE 3

Distribution or retail sales of encapsulated toothpastes according to the invention may be in conventional form - as preferably paper or other biodegradable containers of capsules with the usual admixture of brands and information. Capsules may be sold in single or various shapes, colours, or flavours, and may be sold in various sizes. Variants better suited to humid (tropical) environments, military use, or other rough handling may be sold separately. Retail packs may be of separate items but can include a complete dental hygiene system displayed on a standard blister pack as shown in Fig 6. Here, 600 is the printed laminated card; 601 is an edge of the blister, 602 is a box of evidently blueberry flavoured (604) capsules or beads, 603 is the handle of an accompanying toothbrush including a bead storage container within the handle. The purchaser simply supplies a little water.

A very similar presentation may be used by businesses, airlines or hotels as "complimentary gifts". Ranges of flavours, ranges of capsule sizes, and ranges of toothbrush bristle characteristics may be offered (see Fig 8).

### EXAMPLE 4

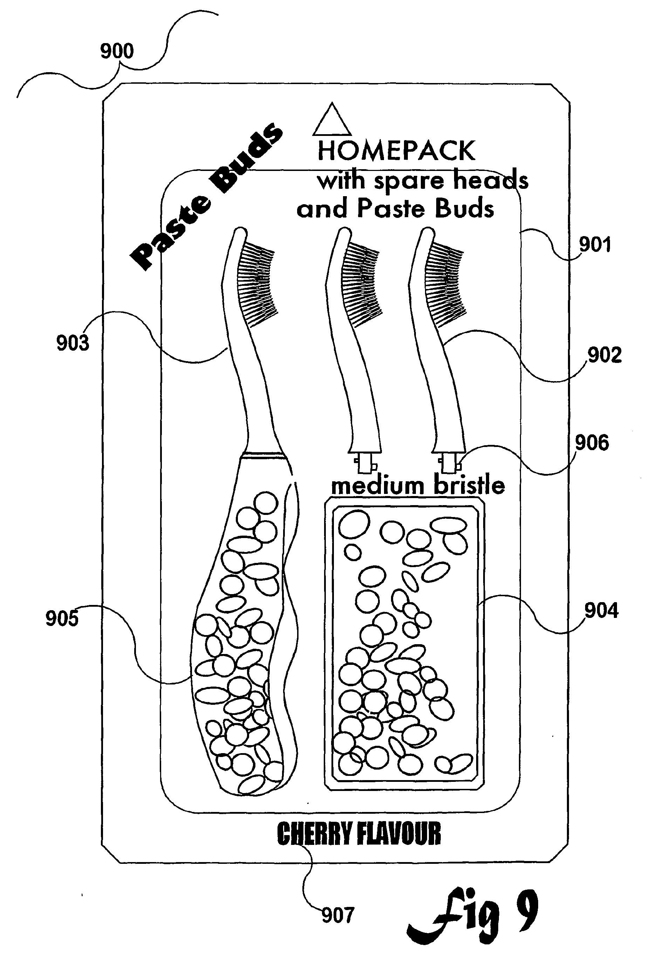

Fig 7 and fig 7B (in closed configuration) shows an example of a toothbrush 700 according to the invention; having a cavity 704, closed by a cap 705 and able to contain a plurality of solid items 106, such as beads of encapsulated toothpaste according to Example 1. The bristles 701 are supported on the usual toothbrush shaft 702, arising from one end of the exterior of the cavity, moulded so as to form a convenient handle 703. A cap 706 protects the bristles. as shown in Fig 7B. When the toothbrush is in use, the cap can serve as a convenient extension to the handle 703 as shown in Fig 7. Note that not all toothbrushes need to have covers; covers are mainly useful for (for example) travellers, or those who carry a toothbrush in the handbag.

The similar toothbrush of Fig 8 (handle portion 703 only shown, in section) includes a two-cavity storage area (cavities 801 and 803) so that different types of object (802 and 804 respectively) may be stored separately within the toothbrush handle, so providing the convenience of this oral hygiene system. The triangular objects 804 may for example be mouthwash, oral disinfectant, or further toothpaste beads 802. Again there is a cap, 705. Recent awareness of the heightened risk of orally transmitted diseases (including meningitis and recently (in the U K) tuberculosis) where kissing, or shared drinking vessels may be involved in transmission leads to the usefulness of a cache of medicated tablets as part of an oral hygiene system according to the invention. The two-container toothbrush (one cavity holding oral hygiene tables; the other holding dental hygiene beads) can be carried anywhere.

Fig 9 shows another example toothbrush, with no cap 706, with spare heads, and spare toothpaste, in a pack ready for sale as a dental hygiene system which includes the toothpaste as encapsulated beads or portions according to the invention. Note that the indicia (e.g. 907, for identification of flavour) included in this figure is solely intended to signify possible advertising statements. In Fig 9, a card 900 includes a blister pack 901 which should ensure effective sterility of the contents prior to opening. 903 indicates a complete toothbrush assembly including some beads of toothpaste in the handle 905. Spare toothpaste beads are included in the box 904. 902 is one of three heads for the toothbrush. It will be appreciated that worn-out toothbrush heads contribute to poor dental hygiene in a population, hence increased availability of spare heads is useful. The spare heads may be of the same bristle stiffness, or may be in different colours or bristle shapes. Each head includes a reversible attachment means such as a bayonet lock 906 capable of interlocking with a corresponding socket in the handle 905. Other attachment means such as a screw thread on the brush head, capable of being fitted to a threaded hole in the top of the handle, are possible alternatives as will be appreciated by a person versed in the mechanical arts. The supply of spare heads reduces total plastics waste. A seal over the cavity within the handle is not shown in this illustration. Nor is access to an optional second cavity as for Fig 8.

Variations on the toothbrush design include those with more than one cavity (where one can be used for storing other items such as breath fresheners, floss, and the like). For electric toothbrushes, battery size is diminishing thanks to improvements in rechargable battery technology, providing space within the handle of an electric toothbrush for a cavity to hold some beads.

### COMMERCIAL ADVANTAGES

As a dental hygiene system, the invention has the notable advantage that it provides an integrated combination of toothbrush and toothpaste, in a form likely to be attractive to users and especially to children. There have not been any significant changes in the presentation of compounds usable as toothpastes/dentifrices for some years. Dentifrices are supplied in powder form in a container. Toothpastes are supplied in paste form in a tube. Yet the dental health of children in particular remains under threat, especially with the availability of sweetened drinks and snack foods in the Western world.

**Advantages of the dental hygiene system of this invention include:**
1. The portability of the oral hygiene system is convenient because a compact article provides all materials and tools required for cleaning the teeth but the water.
2. The oral hygiene system is convenient for travelling people and those undertaking outdoor pursuits such as scouts, climbers, military personnel or the like.
3. Apart from the emphasis on tooth hygiene, a rise in orally transmitted diseases (including meningits and recently tuberculosis) where kissing or shared drinking vessels may be involved i transmission leads to desirability of providing a cache of medicated tablets within an oral hygiene system according to the invention.
4. Touring cyclists, trampers and climbers (for example) find the weight and volume of an entire toothpaste tube a significant extra load and consumer of space; this invention minimises that.
5. Promotional toothbrushes as used by airlines and hotels for example are more conveniently manufactured and distributed together with this more easily handled toothpaste composition. Perhaps as few as 2 - 6 beads per pack need be supplied. Beads may be refilled from dispensers or convenient pocket packs.
6. The invention is compatible with, and likely to enhance existing programmes of tooth care and dental hygiene, especially those for children, where the variety of beads and the control of the amount in each is likely to assist in brushing the teeth.
7. Waste packaging materials such as toothpaste tubes are minimized, so reducing pollution and landfill.
8. Toothbrush design can be adapted for the storage of a number (perhaps only a small number) of beads/capsules within the handle, thereby resulting in a single object for a single function (cleaning the teeth).
9. Even the ubiquitous narrow plastic handled toothbrush can be made with a visible internal cavity and a removable, perhaps resilient resealable plug for accessing a capsule as required - perhaps only in emergency.
10.A toothbrush having a solid, well-made handle with a cavity for containing capsules can be made to use a replaceable brush end, and optionally also can be made with a cover for travelling. The cover may become part of the handle when in use, consequently reducing the bulk to be carried.

Inherent advantages of the beads or capsules of this invention include:
1. Convenience in storage, handling, and use; the material behaves as dry portions until put into the mouth. The beads are easily stored, decanted, and dispensed.
2. Each member of a family may use the flavour and type of toothpaste that they prefer which is an advantage for those trying to encourage their children to clean their teeth voluntarily.
3. the beads may be presented in many forms or styles, particularly including visual attractiveness, varieties in shape and colour, and hence added appeal to children. Children can pick a flavour - like picking a flavoured jellybean from a mixed bag, or picking a shape, like a star for the evening.
4. There is an opportunity to mix flavours, especially for children, within the one pack.
5. A single dispenser may be shared, without hygiene / crossinfection problems, by groups of people (such as a family, or a school camp, scout camp, or military group).
6. Tablets of breath freshener, headache pills, or the like can be stored together with beads of encapsulated toothpaste. (This is particularly useful for a traveller).
7. environmentally friendly manifold reduction in waste plastics.

Finally, it will be understood that the scope of this invention as described and/or illustrated within this provisional specification is not limited to the preferred embodiments described herein for illustrative purposes. Those skilled in the art will appreciate that various modifications, additions, and substitutions are possible without departing from the scope of the invention as set forth in the following claims.

## Claims

1. A system for facilitating the practice of oral and dental hygiene; the system including an oral or dental hygiene composition having a paste-like consistency and provided for use within a plurality of disposable containers or pellets each having a dry exterior wall and each containing about enough composition for a single use, wherein the wall of each pellet is capable of being ruptured by pressure against or between the teeth, or against the bristles of a toothbrush, and that each pellet is provided with distinctive size and/or shape and/or color so that a person intending to perform oral or dental hygiene is able to select a desired type of pellet from a plurality of pellets in a bulk supply, ***characterised in that*** the wall of each pellet has an exterior wall composed of a starch material, so that there is no residual indigestible matter.

2. A system as claimed in claim 1, ***characterised in that*** each pellet is provided with a distinctive shape selected from a range of distinctive shapes, so that a person is able to select a desired type of pellet from a bulk supply.

3. A system as claimed in claim 1, ***characterised in that*** each pellet is provided with a distinctive colour selected from a range of distinctive colours, so that a person is able to select a desired type of pellet from a bulk supply.

4. A system as claimed in claim 1, ***characterised in that*** each pellet is provided with a distinctive flavour selected from a range of distinctive flavours, so that a person is able to select a desired type of pellet from a bulk supply.

5. A system as claimed in claim 1, further comprising a toothbrush and a bulk supply of said oral or dental hygiene composition having a paste-like consistency and a distinctive appearance, which is held ready for use within at least one resealable cavity formed within a handle of a toothbrush; the toothbrush being also provided with a bristle cover capable of protecting the bristles of the toothbrush during carriage or transport so that the system can be carried on one's person as a complete, portable unit.

6. A system as claimed in claim 5, ***characterised in that*** the handle of the toothbrush includes two resealable cavities, so that at least one cavity is available for storage and dispensing of an oral hygiene composition other than a dental hygiene composition.

## Patentansprüche

1. System zur Erleichterung der Ausübung von Oral- und Dentalhygiene; das System umfassend eine Oral- oder Dentalhygiene-Zusammensetzung, die eine pastenartige Konsistenz hat und zur Verwendung in einer Vielzahl von Einwegbehältnissen oder Kapseln vorgesehen ist, die jeweils eine trockene Außenwandung haben und jeweils in etwa ausreichend Zusammensetzung für einen einmaligen Gebrauch enthalten, worin die Wandung einer jeden Kapsel durch Druck gegen die oder zwischen den Zähnen oder gegen die Borsten einer Zahnbürste zerbrochen werden kann, und dass jede Kugel mit ausgeprägten Merkmalen versehen ist, die aus einer Auswahl von ausgeprägter Größe und/oder Form und/oder Farbe ausgewählt sind, so dass eine Person, die Oral- oder Dentalhygiene durchführen will, einen erwünschten Kapseltyp aus einer Vielzahl von Kapseln in einer Schüttzufuhr auswählen kann, **dadurch gekennzeichnet, dass** die Wandung einer jeden Kapsel eine, sich aus Stärkematerial zusammensetzende Außenwandung hat, so dass es kein unverdauliches Restmaterial gibt.

2. System nach Patentanspruch 1, **dadurch gekennzeichnet, dass** jede Kapsel mit einer ausgeprägten Form versehen ist, die aus einer Auswahl ausgeprägter Formen ausgewählt ist, so dass eine Person einen erwünschten Kapseltyp aus einer Schüttzufuhr auswählen kann.

3. System nach Patentanspruch 1, **dadurch gekennzeichnet, dass** jede Kugel mit einer ausgeprägten Farbe versehen ist, die aus einer Auswahl ausgeprägter Farben ausgewählt ist, so dass eine Person einen erwünschten Kapseltyp aus einer Schüttzufuhr auswählen kann.

4. System nach Patentanspruch 1, **dadurch gekennzeichnet, dass** jede Kugel mit einem ausgeprägten Geschmack versehen ist, der aus einer Auswahl ausgeprägter Geschmäcke ausgewählt ist, so dass eine Person einen erwünschten Kapseltyp aus einer Schüttzufuhr auswählen kann.

5. System nach Patentanspruch 1, des Weiteren umfassend eine Zahnbürste und eine Schüttzufuhr der Oral- oder Dentalhygiene-Zusammensetzung, die eine pastenartige Konsistenz und ein ausgeprägtes Aussehen hat, die in zumindest einem wiederverschließbaren Hohlraum gebrauchsfertig gehalten wird, der im Griff einer Zahnbürste ausgebildet ist; welche Zahnbürste auch mit einer Borstenabdeckung versehen ist, die die Borsten der Zahnbürste bei Beförderung oder Transport derart schützen kann, dass das System als vollständige, tragbare Einheit mitgenommen werden kann.

6. System nach Patentanspruch 5, **dadurch gekennzeichnet, dass** der Griff der Zahnbürste zwei wiederverschließbare Hohlräume umfasst, wobei zumindest ein Hohlraum zur Lagerung und Dispensierung einer anderen Mundhygiene-Zusammensetzung als einer Dentalhygiene-Zusammensetzung zur Verfügung steht.

## Revendications

1. Système pour faciliter la pratique de l'hygiène buccale et dentaire; le système comprend un mélange pour l'hygiène buccale et dentaire ayant une consistance du type dentifrice et prévu pour être utilisé dans une pluralité de conteneurs jetables ou de pastilles qui ont une paroi extérieure sèche et chacun de ceux-là contenant suffisamment de ce mélange pour un usage unique, dans lequel système la paroi de chaque pastille peut être cassée par pression contre ou entre les dents, ou contre les poils d'une brosse à dents, et que chaque pastille est **caractérisée par** une gamme de tailles et/ou de formes et/ou de couleurs différentes de manière qu'une personne qui a l'intention de procéder à l'hygiène buccale ou dentaire puissent choisir le type de pastille de son choix parmi une pluralité de pastilles dans un distributeur en vrac, **caractérisé en ce que** la paroi de chaque pastille a une paroi extérieure composée d'amidon de sorte qu'il ne reste aucune matière résiduelle indigestible.

2. Système selon la revendication 1, **caractérisé en ce que** chaque pastille est dotée d'une forme différente choisie dans une gamme de formes distinctives de manière que chaque personne puisse choisir le type de pastille voulu dans une quantité en vrac.

3. Système selon la revendication 1, **caractérisé en ce que** chaque la pastille est dotée d'une couleur différente choisie dans une gamme de couleurs distinctives de manière qu'une personne puisse choisir le type de pastille voulu dans une quantité en vrac.

4. Système selon la revendication 1, **caractérisé en ce que** chaque la pastille a un goût différent choisi dans une gamme de goûts différents, de manière qu'une personne puisse choisir le type de pastille voulu dans une quantité en vrac.

5. Système selon la revendication 1, comprenant en plus une brosse à dents et un distributeur en vrac dudit mélange pour l'hygiène buccale et dentaire ayant une consistance du type dentifrice et une apparence distinctive, lequel mélange est maintenu prêt pour utilisation dans au moins une cavité pouvant être refermée formée à l'intérieur du manche d'une brosse à dents; la brosse à dents étant également munie d'un capuchon pour protéger les poils de la brosse pendant le transport de sorte que l'on puisse prendre le système avec soi comme une unité portable complète.

6. Système selon la revendication 5, **caractérisé en ce que** le manche de la brosse à dents comprend deux cavités pouvant être refermées, de manière qu'au moins une cavité soit disponible pour stocker et distribuer un mélange pour l'hygiène buccale autre qu'un mélange pour l'hygiène dentaire.
